# EUROPEAN PATENT APPLICATION

(11) **EP 4 796 157 A1**
(43) Date of publication of application: **26.08.2026**
(21) Application number: 23959325.4
(22) Date of filing: 05.12.2023
(51) Int. Cl.: A61M 37/00

(54) **SKIN TREATMENT ELEMENT**

(30) Priority: 20.11.2023 CN 202311543685
(71) Applicant: Suzhou Natong Biomed Co., Ltd, Jiangsu 215000 (CN)
(72) Inventor: XU, Bai, Suzhou, Jiangsu 215000 (CN)
(74) Representative: Ipsilon
(86) International application number: PCT/CN2023/136358
(87) International publication number: WO 2025/107358

(57) **Abstract**

Provided is a skin treatment element with a microstructure. The skin treatment element includes a substrate (1) and treatment units (2). The treatment unit (2) has a treatment end (21) and a connection seat (22). The connection seat (22) is connected to one side surface of the substrate (1). The treatment end (21) has a first blade portion (211). The skin treatment element can better balance the structural strength and piercing capability of the treatment unit, and reduce stringent requirements on materials and manufacturing processes of the product, thereby lowering the overall production cost, improving the safety of the product, and enabling the microneedle transdermal drug delivery product to serve the public more widely.

## Description

### TECHNICAL FIELD

The present disclosure relates to a skin treatment tool used in the field of transdermal drug delivery technologies, and in particular, belongs to a skin treatment element having a microstructure.

### BACKGROUND

The stratum corneum of human skin functions as a protective barrier; however, it also makes transdermal absorption of active ingredients difficult. In the field of transdermal drug delivery technologies, a chemical method or a physical method may be employed to improve the transdermal absorption efficiency of the active ingredients. A conventional product relies on the chemical method to improve the transdermal absorption efficiency, with plasters as a common form. However, a chemical penetration enhancer incorporated in the plasters tends to cause skin redness, itching, and allergic reactions, leading to certain drawbacks. A new transdermal delivery method adopts the physical method. Among products in which the transdermal absorption efficiency is improved via the physical method, a microneedle transdermal drug delivery product features advantages such as safety, high efficiency, and ease of operation, and thus is favored by experts both domestically and internationally.

The microneedle transdermal drug delivery product is made of metal, monocrystalline silicon, polymers, or other suitable materials, and the microneedle transdermal drug delivery product includes a substrate and a microneedle unit array formed on the substrate. For example, such a technical solution is disclosed in the Chinese granted invention patent ''metal microneedle array chip and preparation method and use thereof'' (Granted Patent Publication No.: CN100402107C). The microneedle transdermal drug delivery product acts on a skin surface through the microneedle unit array, whereby the barrier layer of the skin surface is overcome, and a dredging channel is formed, thereby enabling rapid and efficient transdermal absorption of the active ingredients. It exhibits the advantages of minimal invasiveness, non-invasiveness, painlessness, safety, and high efficiency. The microneedle transdermal drug delivery product may be used in conjunction with various medical devices and may have broad application prospects, such as the technical solution and related applications disclosed in the Chinese granted invention patent ''built-in non-verbal instruction device integrable into a drug applicator'' (Granted Patent Publication No.: CN103079634B).

However, an existing microneedle transdermal drug delivery product has many aspects to be improved.

First, in the existing microneedle transdermal drug delivery product, the width of a microneedle unit is on the micrometer-scale or even the nanometer-scale. The needle body is generally conical and is sharply tapered, with an aspect ratio typically being 5 or even 10 so as to ensure that the product has good piercing capability. However, the microneedle unit with this structure is prone to fracture and breakage during processing, which results in low product yield and correspondingly high processing costs, and thus makes the product inaccessible to ordinary patients.

Second, during the use of the microneedle transdermal drug delivery product, microneedle units may break off and remain embedded in the skin; therefore, the safety of this product needs to be further improved.

In summary, the current microneedle transdermal drug delivery product suffers from various technical problems, including insufficient structural strength, low production yield, high production costs, and insufficient safety in use.

### SUMMARY

### Technical problem

The current microneedle transdermal drug delivery product suffers from various technical problems, including insufficient structural strength, low production yield, high production costs, and insufficient safety in use.

### Technical solution

In view of the various technical problems of the current microneedle transdermal drug delivery product, such as insufficient structural strength, low production yield, high processing costs, and insufficient safety in use, the present disclosure discloses a skin treatment element.

The skin treatment element includes a substrate and treatment units. The treatment unit includes a treatment end and a connection seat. The connection seat is connected to one side surface of the substrate. The treatment end includes a first blade portion.

Preferably, the first blade portion is parallel to the substrate.

Preferably, an extension line of the first blade portion intersects the substrate.

Preferably, the extension line of the first blade portion intersects the substrate at an included angle, and the included angle is greater than 0° and not greater than 60°.

Preferably, the first blade portion has a length of not less than 10 nanometers.

Preferably, the substrate is made of at least one or a combination of a monocrystalline silicon material, a polymer material, a metal material, or plastic.

Preferably, when the substrate is made of the monocrystalline silicon material, the substrate has a crystal plane orientation selected from (100), (110), and (111).

Preferably, one side surface of the substrate is formed with a columnar base material, and the treatment units are formed by etching the columnar base material through a physical method and a chemical method.

Preferably, the columnar base material has a shape of a polygonal prism or a cylinder, and an outer contour of the connection seat is polygonal or circular.

Preferably, the columnar base material has a shape of a quadrangular prism, a hexagonal prism, or an octagonal prism, and the outer contour of the connection seat is rectangular, hexagonal, or octagonal.

Preferably, the columnar base material is etched by the physical method and the chemical method along the crystal plane orientation to form an etched plane.

Preferably, the treatment unit is provided with at least two etched planes, and the at least two etched planes are formed between the connection seat and the treatment end.

Preferably, the treatment end includes a second blade portion, the second blade portion and the first blade portion are distributed at an edge of the same etched plane among the at least two etched planes, and the first blade portion and the second blade portion are disconnected.

Preferably, the treatment end includes a second blade portion, the second blade portion is connected end-to-end to the first blade portion, and an included angle is formed between the second blade portion and the first blade portion.

Preferably, the treatment units are made of at least one of a monocrystalline silicon material, a polymer material, or a metal material.

Preferably, the treatment units are arranged on one side surface of the substrate in an array.

Preferably, the treatment units are arranged on the substrate in a grid-like array.

Preferably, a spacing between adjacent treatment units among the treatment units is not less than 0.1 mm and not greater than 5 mm.

Preferably, the treatment unit has a height of not less than 10 nanometers and not greater than 1 millimeter.

Further explanation of the above technical solution is as follows.

In the present application, the substrate may be made of the monocrystalline silicon material, the metal material, the polymer material, and the like, and the processing flow includes a pretreatment operation and a forming operation. In the pretreatment operation, pretreatment is performed on the substrate through a corresponding manufacturing process according to different substrate materials, so as to form the columnar base material on one side surface of the substrate. In the forming operation, the structural morphology of the columnar base material is modified, thereby finally forming the treatment unit. By using the monocrystalline silicon material as an example, monocrystalline silicon forming the substrate may have the crystal plane orientation selected from (100), (110), and (111). Pretreatment operations such as masking, photolithography, and etching may be performed on the substrate, so that the columnar base material having a structural shape such as a polygonal prism or a cylinder is formed on one side surface of the substrate. After pretreatment, the columnar base material may be processed through a 3D etching process to finally form the treatment unit. It is to be noted that the 3D etching process includes physical and chemical etching processes, such as a chemical anisotropic etching process, a dry etching process, and a wet etching process.

For the connection seat and the treatment end, the treatment unit is formed on the substrate and extends outward and grows from one side surface of the substrate. One end of the treatment unit connected to the substrate is defined as the connection seat, and one end of the treatment unit facing away from the substrate is defined as the treatment end. The treatment end is configured to contact the skin and form a dredging channel on the skin surface.

For the etched plane, the monocrystalline silicon has multiple different crystal plane orientations, such as (100), (110), and (111), and the monocrystalline silicon exhibits anisotropy. When anisotropic etching is performed on the monocrystalline silicon by using the chemical method, it exhibits different etching rates on different crystal planes. In the present application, the treatment unit may be formed by subjecting the columnar base material to the 3D etching process, and chemical anisotropic etching is performed on the columnar base material during the processing. In the same etching process, the columnar base material has multiple different crystal plane orientations with different etching rates; therefore, multiple "etched planes" with different angles and directions are formed on the columnar base material.

For the blade portion, the treatment unit undergoes etching in different directions during processing, thereby forming multiple etched planes with different angles and directions on the surface of the treatment unit. An edge where these etched planes intersect forms a sharp edge, which is defined as the "blade portion". A blade portion located at an end of the treatment end, and the blade portion capable of first contacting the skin is defined as a "first blade portion", and a blade portion adjacent to the "first blade portion" is defined as a "second blade portion".

For the length of the blade portion, an edge where these etched planes intersect forms a sharp edge, the sharp edge is defined as the "blade portion", and the length of the sharp edge is defined as "the length of the blade portion". Correspondingly, the "blade portion" also has a thickness, and "the thickness of the blade portion" refers to the thickness of the edge.

The first blade portion and the second blade portion are disconnected. In some examples, the first blade portion and the second blade portion are located at two edges of the same etched plane, such as two opposite sides of a rhombic etched plane, and in this case, the first blade portion and the second blade portion are disconnected. During operation of the treatment unit, the first blade portion first contacts the skin, then the etched plane on the treatment end slides relative to the skin, and subsequently the second blade portion contacts the skin.

The included angle is formed between the first blade portion and the second blade portion. In some examples, the first blade portion may be formed by two etched planes with different directions. On this basis, a third etched plane (with a direction different from the previous two etched planes) etches the treatment end and removes part of the first blade portion. In this case, an edge of the third etched plane forms the second blade portion, and the second blade portion is connected to the first blade portion to form a certain included angle. During operation of the treatment unit, the first blade portion first contacts the skin, and the second blade portion subsequently contacts the skin.

For the included angle between the extension line of the first blade portion and the substrate, the "first blade portion" is formed during the etching processing of the treatment unit. Due to different etching directions, the extension line of the "first blade portion" may be parallel to the substrate (analogous to a "wedge shape") or form a certain angle with the substrate (analogous to the "end of a Tang sword"). The magnitude of this angle may be varied by adjusting the etching direction.

### Beneficial effects

In this technical solution, the treatment end of the treatment unit is provided with a thin and sharp first blade portion. Micron-scale or even nanometer-scale precision can be achieved through etching processing, which satisfies the skin dredging and care requirements, and enables the formation of micron-scale or nanometer-scale dredging channels on the skin surface so as to facilitate efficient transdermal absorption of effective ingredients. The technical solution is distinctive in that the first blade portion has a sufficient dimension in the length direction, so that the overall structure of the treatment unit is more reliable and robust, thereby effectively improving the yield of the treatment unit during processing, and reducing the risk of breakage when the treatment unit treats the skin. In summary, the skin treatment element in this technical solution can better balance the structural strength and piercing capability of the treatment unit, and reduce stringent requirements on materials and manufacturing processes of the product, thereby lowering the overall production cost, improving the safety of the product, and enabling the microneedle transdermal drug delivery product to serve the public more widely.

### BRIEF DESCRIPTION OF THE DRAWINGS

FIG. 1 is an enlarged schematic view of a treatment unit in a skin treatment element according to an embodiment one of the present disclosure.
FIG. 2 is a schematic view of a skin treatment element according to an embodiment one of the present disclosure.
FIG. 3 is a schematic view of a skin treatment element acting on a skin stratum corneum according to an embodiment one of the present disclosure.
FIG. 4 is an enlarged schematic view of a treatment unit in a skin treatment element according to an embodiment two of the present disclosure.
FIG. 5 is an enlarged schematic view of a treatment unit in a skin treatment element according to an embodiment three of the present disclosure.
FIG. 6 is a schematic view of a skin treatment element according to an embodiment three of the present disclosure.
FIG. 7 is an enlarged schematic view of a treatment unit in a skin treatment element according to an embodiment four of the present disclosure.
FIG. 8 is an enlarged schematic view of a treatment unit in a skin treatment element according to an embodiment five of the present disclosure.
FIG. 9 is a schematic view of a skin treatment element according to an embodiment five of the present disclosure.
FIG. 10 is an enlarged schematic view of a treatment unit in a skin treatment element according to an embodiment six of the present disclosure.
FIG. 11 is an enlarged schematic view of a treatment unit in a skin treatment element according to an embodiment seven of the present disclosure.
FIG. 12 is a schematic view of a skin treatment element according to an embodiment seven of the present disclosure.
FIG. 13 is a top-view enlarged electron micrograph of a treatment end in a skin treatment element according to an embodiment eight of the present disclosure.

### List of reference numbers

- T: stratum corneum
- Y: skin treatment element
- 1: substrate
- 2: treatment unit
- 21: treatment end
- 211: first blade portion
- 212: second blade portion
- 213: third blade portion
- 22: connection seat
- 23: etched plane

### DETAILED DESCRIPTION

Preferred embodiments of the present disclosure will be described below in conjunction with the accompanying drawings. It is to be understood by those skilled in the art that these embodiments are merely used to explain the technical principles of the present disclosure and are not intended to limit the scope of protection of the present disclosure.

A substrate may be made of one or more materials selected from a monocrystalline silicon material, a metal material, a plastic material, a silica gel material, and a polymer material. In some specific embodiments of the present application, the processing flow includes a pretreatment operation and a forming operation. In the pretreatment operation, pretreatment is performed on the substrate through a corresponding manufacturing process according to different substrate materials, so as to form a columnar base material on one side surface of the substrate. In the forming operation, the structural morphology of the columnar base material is processed and modified, thereby finally forming a treatment unit having a treatment end. By using the monocrystalline silicon material as an example, monocrystalline silicon forming the substrate may have a crystal plane orientation selected from (100), (110), and (111). Pretreatment operations such as masking, photolithography, and etching may be performed on the substrate, so that a columnar base material having a structure such as a polygonal prism or a cylinder is formed on one side surface of the substrate. After pretreatment, in the forming operation, the columnar base material may be processed through a 3D etching process. The columnar base material forms multiple etched planes with different directions and angles after physical and chemical etching, and edges between the etched planes finally form blade portions.

In the present application, the skin treatment element Y has a substrate 1 and treatment units 2. The treatment unit 2 has a treatment end 21 and a connection seat 22. The connection seat 22 is connected to one side surface of the substrate 1, and the treatment end 21 has a first blade portion 211.

The shape of the substrate 1 may be adaptively cut according to the contour of an adapter or a platform mounted on an instrument, such as rectangular, square, regular hexagonal, regular octagonal, circular, and the like. In some application scenarios, the thickness of the substrate 1 is not less than 0.1 mm, such as 0.1 mm, 0.15 mm, and 0.2 mm.

The treatment unit 2 may be made of one or more materials selected from a monocrystalline silicon material, a metal material, a plastic material, a silica gel material, and a polymer material. In some embodiments, the treatment unit 2 and the substrate 1 may be made of the same material, for example, the treatment unit 2 and the substrate 1 are made of the monocrystalline silicon material or the polymer material. In some embodiments, the treatment unit 2 and the substrate 1 may be made of different materials, for example, the treatment unit 2 is made of the polymer material, and the substrate 1 is made of the monocrystalline silicon material or the metal material. In some application scenarios, the height of the treatment unit 2 ranges from 10 nanometers to 1 millimeter, such as 20 nm, 50 nm, 80 nm, 100 nm, 200 nm, 300 nm, 500 nm, 10 µm, 20 µm, 50 µm, and 200 µm.

The skin treatment element Y may be used in conjunction with various medical devices, such as a massager, a transdermal delivery device, a water mesotherapy machine, and a roller, and thus has broad application prospects. The skin treatment element Y has various styles in different application scenarios, which will be further described through several embodiments below.

### Embodiment one

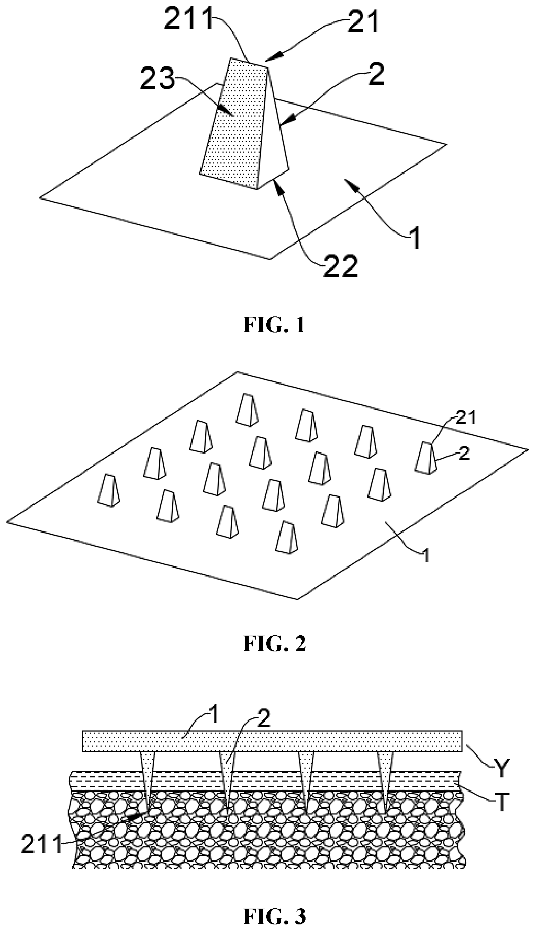

FIG. 1 is an enlarged schematic view of a treatment unit in a skin treatment element according to an embodiment one of the present disclosure. FIG. 2 is a schematic view of a skin treatment element according to an embodiment one of the present disclosure. FIG. 3 is a schematic view of a skin treatment element acting on a skin stratum corneum according to an embodiment one of the present disclosure. As shown in FIGS. 1 and 2, in embodiment one, the treatment units 2 are arranged in an array on one side surface of the substrate 1 (the thickness of the substrate 1 is omitted for simplicity), with a specific arrangement specification of 4 × 4. It is readily conceivable that the treatment units 2 may also adopt other suitable arrangement specifications according to design requirements, such as square matrix arrangement specifications including 3 × 3, 5 × 5, 6 × 6, 7 × 7, 8 × 8, 9 × 9, 10 × 10, and rectangular matrix arrangement specifications including 3 × 4, 4 × 5, 4 × 6, and other suitable specifications may be employed. It is readily understandable that the treatment units 2 may also be arranged in a honeycomb array, a concentric circular array, or other suitable arrays. The specific arrangement and specifications may be flexibly adjusted according to user requirements and application scenarios, and this embodiment is not intended to be limiting. In embodiment one, the spacing between adjacent treatment units 2 is not less than 0.1 mm and not greater than 5 mm. By adjusting the arrangement interval of the treatment units 2, the treatment efficiency of the skin treatment element on the skin may be varied to satisfy different care requirements. Optional dimensions include, for example, 0.35 mm, 0.4 mm, or 0.46 mm.

The treatment unit 2 is fixedly connected to the substrate 1 through the connection seat 22. The treatment unit 2 may be formed by subjecting the columnar base material to the 3D etching process. After physical and chemical etching, the treatment end 21 is formed at the end of the treatment unit 2 facing away from the substrate 1, and the treatment unit 2 has multiple etched planes 23.

As shown in FIGS. 1 and 2, in embodiment one, the outer contour of the connection seat 22 is rectangular. Alternatively, the outer contour of the connection seat 2 may be processed into a polygonal or circular shape. Accordingly, the columnar substrate may be a prism or a cylinder, such as a quadrangular prism, hexagonal prism, or octagonal prism. By using the columnar substrate as the quadrangular prism, the treatment unit 2 in embodiment one forms two etched planes 23 after etching. The etching process is performed from the treatment end 21 down to the connection seat 22, thereby forming etched planes 23 extending from the treatment end 21 to the connection seat 22 on the treatment unit 2, as shown by the shaded region in FIG. 1 (one etched plane is obscured). After etching, the treatment unit 2 is formed into a wedge shape. The tip of the wedge (i.e., an edge where the two etched planes 23 intersect) forms a sharp ridge, which constitutes the first blade portion 211 of the treatment end 21. During processing, a sharp and thin blade portion can be fabricated by controlling the etching direction, thereby improving the dredging treatment effect of the treatment unit 2 on the skin. In embodiment one, the first blade portion 211 is parallel to the substrate 1. The length of the first blade portion 211 is not less than 10 nm, such as 10 nm, 100 nm, 500 nm, 1000 nm, or 8000 nm. Further, the length of the first blade portion 211 may be not less than 500 nm or not less than 1000 nm. This structure enables the treatment unit 2 to have a sufficiently large and thick dimension in the length direction of the blade portion, thereby effectively improving the structural strength and reliability of the treatment unit, and further increasing the yield during the etching process.

It is to be noted that in some other embodiments, the etching process may be performed only on the treatment end 21, that is, the etched plane 23 does not extend to the connection seat 22. The blade portion may still be formed on the treatment end 21, and the treatment unit with such a structure can also achieve the corresponding transdermal delivery care function. It is also to be noted that the treatment unit 2 may also use the octagonal prism as the substrate, with the treatment end formed by etching.

The skin treatment element Y in embodiment one is used with the transdermal delivery device. The transdermal delivery device drives the skin treatment element Y to vibrate reciprocally at high frequency, thereby performing a dredging treatment on the skin surface. As shown in FIG. 3, the first blade portion 211 on the treatment unit 2 can easily pierce into the stratum corneum T of the skin, so as to form the dredging channel on the stratum corneum T, thereby facilitating efficient transdermal absorption of active ingredients. Moreover, as shown in FIG. 2, the first blade portion 211 has a relatively large length in the length; therefore, the first blade portion 211 possesses sufficient structural strength, thereby further improving the safety of the product.

### Embodiment two

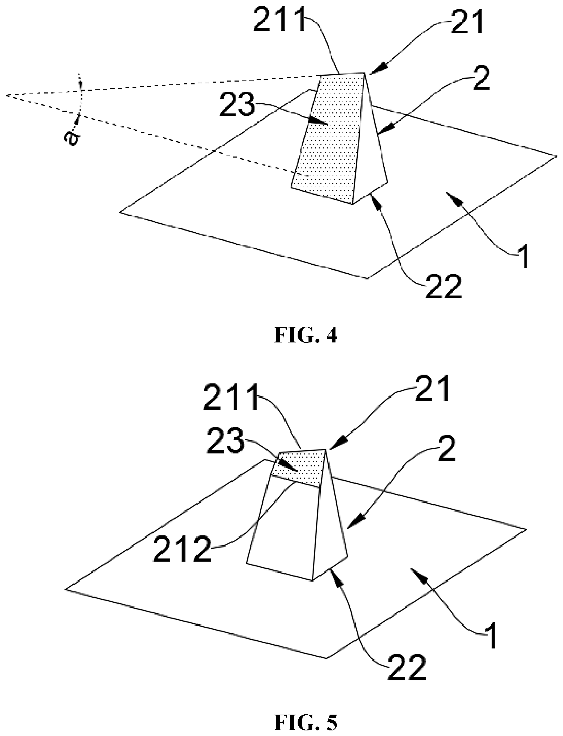

FIG. 4 is an enlarged schematic view of a treatment unit in a skin treatment element according to an embodiment two of the present disclosure. As shown in FIG. 4, in embodiment two, unlike embodiment one, the two etched planes 23 have different angles and directions, so that the formed first blade portion 211 presents a certain inclined angle, that is, an extension line of the first blade portion 211 intersects the substrate 1. The included angle a is shown by the dashed line in FIG. 4. Optionally, the included angle a in which the extension line of the first blade portion 211 intersects the substrate 1 is greater than 0° and not greater than 60°, such as 10°, 15°, 30°, 45°, and 60°. When the skin treatment element Y in embodiment two is used for caring the stratum corneum, one end of the first blade portion 211 on the treatment unit 2 first contacts the skin, and then the first blade portion gradually pierces into the skin to perform dredging treatment on the skin. The first blade portion with the inclined angle facilitates dredging the stratum corneum and forming the dredging channel.

### Embodiment three

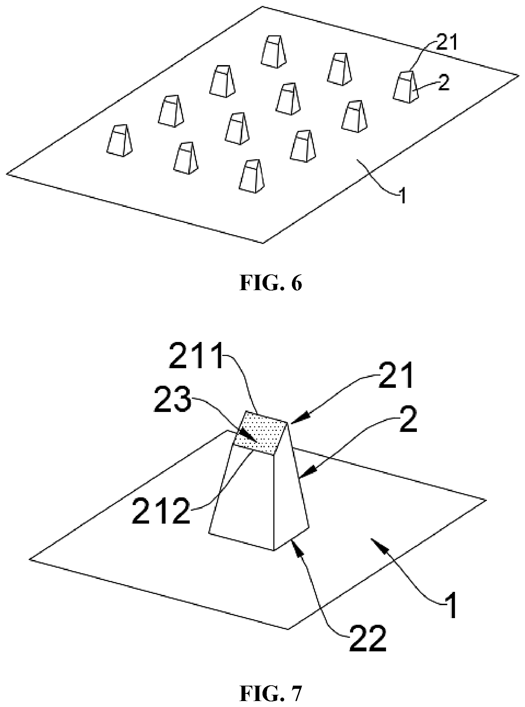

FIG. 5 is an enlarged schematic view of a treatment unit in a skin treatment element according to an embodiment three of the present disclosure. FIG. 6 is a schematic view of a skin treatment element according to an embodiment three of the present disclosure. As shown in FIGS. 5 and 6, in embodiment three, the extension line of the first blade portion 211 intersects the substrate 1. Unlike embodiment two, the treatment unit 2 further includes a second blade portion 212. The second blade portion 212 and the first blade portion 211 are located at two opposite edges of the same etched plane 23, and in this case, the first blade portion 211 and the second blade portion 212 are disconnected. In some cases, the first blade portion 211 is parallel to the second blade portion 212; alternatively, the first blade portion 211 may be non-parallel to the second blade portion 212. As shown in FIG. 6, in embodiment three, the treatment units 2 are arranged in an array of 3 × 4 on the substrate 1. When the skin treatment element Y in embodiment three is used for caring the stratum corneum, one end of the first blade portion 211 on the treatment unit 2 first contacts the skin, and then the first blade portion 211 gradually pierces into the skin. During gradual piercing, the etched plane 23 squeezes the stratum corneum and forms the dredging channel on the skin surface; afterwards, the second blade portion 212 further squeezes the stratum corneum, thereby promoting further opening of the dredging channel and improving the skin absorption effect.

### Embodiment four

FIG. 7 is an enlarged schematic view of a treatment unit in a skin treatment element according to an embodiment four of the present disclosure. As shown in FIG. 7, in embodiment four, the first blade portion 211 is parallel to the substrate 1. Unlike embodiment one, the treatment unit 2 further includes a second blade portion 212. The second blade portion 212 and the first blade portion 211 are located at two opposite edges of the same etched plane 23, and in this case, the first blade portion 211 and the second blade portion 212 are disconnected. When the skin treatment element Y in embodiment four is used for caring the stratum corneum, the first blade portion 211 on the treatment unit 2 first contacts and pierces into the skin. During gradual piercing, the etched plane 23 undergoes relative squeezing against the stratum corneum; in this case, the second blade portion 212 further squeezes the stratum corneum, thereby promoting further opening of the dredging channel and improving the skin absorption effect.

### Embodiment five

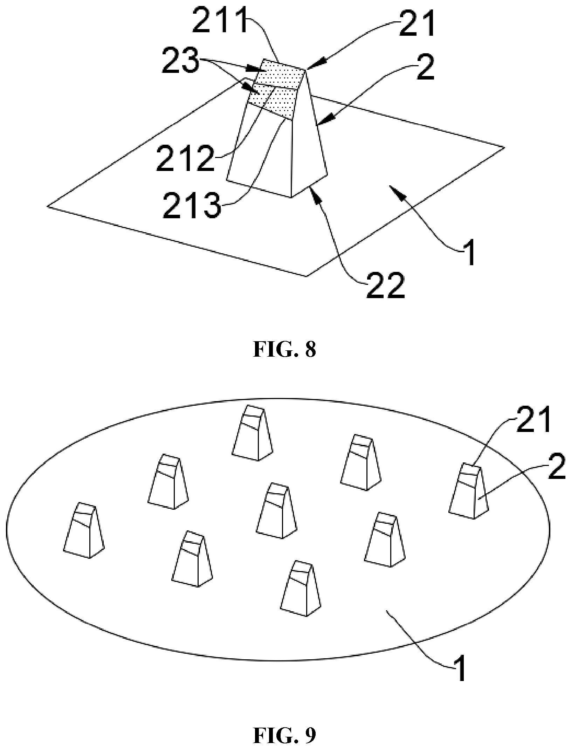

FIG. 8 is an enlarged schematic view of a treatment unit in a skin treatment element according to an embodiment five of the present disclosure. FIG. 9 is a schematic view of a skin treatment element according to an embodiment five of the present disclosure. As shown in FIGS. 8 and 9, in embodiment five, the treatment unit 2 has the first blade portion 211 and the second blade portion 212. Unlike embodiment four, the treatment unit 2 further has a third blade portion 213. The third blade portion 213 and the second blade portion 212 are located at two opposite edges of the same etched plane 23, and in this case, the second blade portion 212 and the third blade portion 213 are disconnected. The first blade portion 211, the second blade portion 212, and the third blade portion 213 form three cutting regions separated from one another. In embodiment five, the substrate 1 is disc-shaped, and the treatment units 2 are arranged in an array of 3 × 3 on one side surface of the substrate 1. The treatment end, which has multiple blade portions and is formed by etching, can not only perform multi-stage treatment on the stratum corneum of the skin surface, thereby effectively promoting further opening of the dredging channel, but also provide good structural strength, thereby reducing the breakage rate of the skin treatment element during processing and improving the safety and reliability of the skin treatment element during skin care.

### Embodiment six

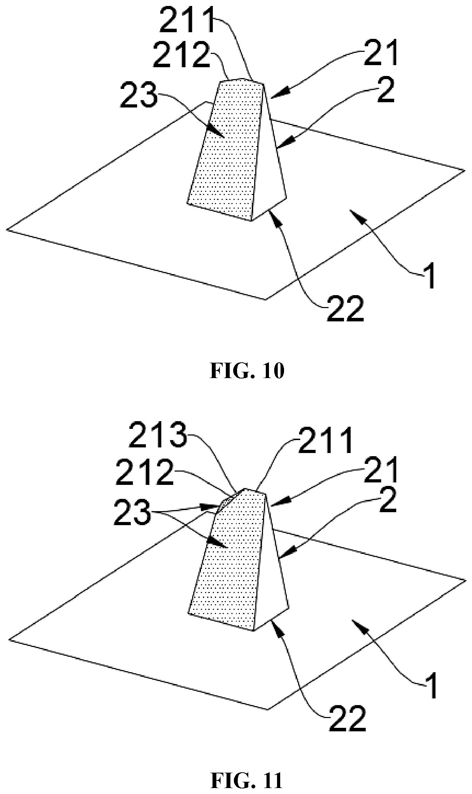

FIG. 10 is an enlarged schematic view of a treatment unit in a skin treatment element according to an embodiment six of the present disclosure. As shown in FIG. 10, in embodiment six, the first blade portion 211 is parallel to the substrate 1. Unlike embodiment one, the treatment unit 2 further includes a second blade portion 212. The second blade portion 212 is connected end-to-end to the first blade portion 211, so that a continuous blade portion with a certain included angle is formed.

### Embodiment seven

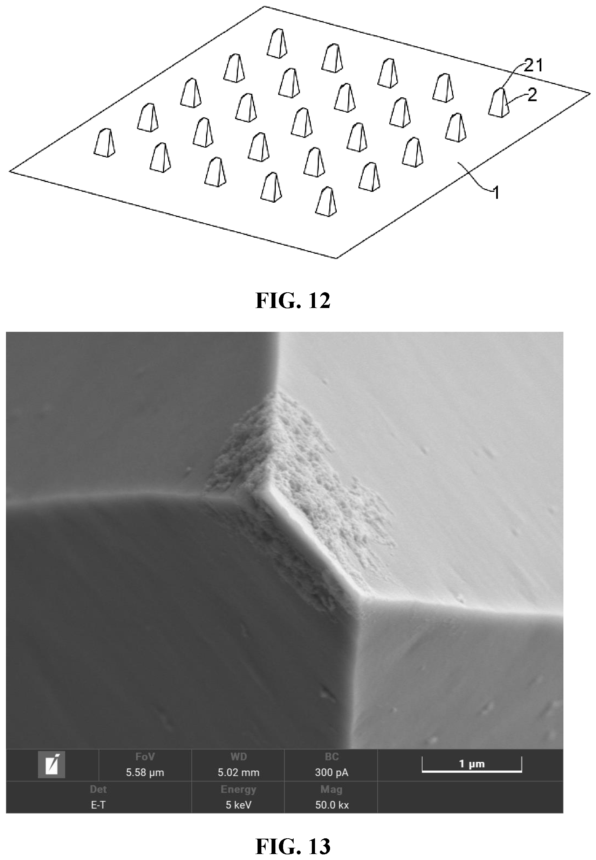

FIG. 11 is an enlarged schematic view of a treatment unit in a skin treatment element according to an embodiment seven of the present disclosure. FIG. 12 is a schematic view of a skin treatment element according to an embodiment seven of the present disclosure. In embodiment seven, the two etched planes 23 have different inclination angles and orientations, the edges of the two etched planes form the first blade portion 211, and the first blade portion 211 is parallel to the substrate 1. Unlike embodiment one, during processing, the first blade portion 211 is re-etched in an etching step, and part of the first blade portion 211 is etched away to form a third etched plane 23. Edges of the third etched plane 23 connected to the first blade portion 211 form the second blade portion 212 and the third blade portion 213, and a certain included angle is formed between the second blade portion 212 and the third blade portion 213. In embodiment seven, the treatment units are arranged in an array of 5 × 5 on one side surface of the substrate 1. When the skin treatment element Y in embodiment seven is used for caring the stratum corneum, the first blade portion 211 on the treatment unit 2 first contacts the skin. During gradual piercing, the three etched planes push the stratum corneum, so that a special dredging channel is formed on the stratum corneum, thereby improving the skin absorption effect.

### Embodiment eight

FIG. 13 is a top-view enlarged electron micrograph of a treatment end in a skin treatment element according to an embodiment eight of the present disclosure. As shown in FIG. 13, in embodiment eight, the treatment end has four etched planes with different directions and angles. Edges between the etched planes form the first blade portion and four second blade portions, which are connected to the first blade portion and have certain included angles. The first blade portion may be parallel to the substrate or form a certain included angle with the substrate.

It is to be additionally noted that the shape and thickness of the substrate 1 may be flexibly adjusted according to design requirements and assembly requirements, the shape of the connection seat 22 on the treatment unit 2 may be adaptively changed according to process requirements or process flows, the number of etched planes 23 may be increased or decreased according to precision requirements and process requirements, and the number and angles of blade portions will also vary with the inclination angles and directions of the etched planes 23, not limited to three or more. All the above modifications in specific details each include a unique blade-type treatment unit and shall fall within the inventive concept of the skin treatment element claimed in the present application.

## Claims

1. A skin treatment element, comprising a substrate and treatment units, wherein a treatment unit of the treatment units comprises a treatment end and a connection seat, and the connection seat is connected to one side surface of the substrate, and the treatment end is provided with a first blade portion.

2. The skin treatment element of claim 1, wherein the first blade portion is parallel to the substrate.

3. The skin treatment element of claim 1, wherein an extension line of the first blade portion intersects the substrate.

4. The skin treatment element of claim 3, wherein the extension line of the first blade portion intersects the substrate at an included angle, and the included angle is greater than 0° and not greater than 60°.

5. The skin treatment element of claim 1, wherein the first blade portion has a length of not less than 10 nanometers.

6. The skin treatment element of claim 1, wherein the substrate is made of at least one or a combination of a monocrystalline silicon material, a polymer material, a metal material, or plastic.

7. The skin treatment element of claim 6, wherein when the substrate is made of the monocrystalline silicon material, the substrate has a crystal plane orientation selected from (100), (110), and (111).

8. The skin treatment element of claim 7, wherein one side surface of the substrate is formed with a columnar base material, and the treatment units are formed from the columnar base material by a chemical anisotropic etching process.

9. The skin treatment element of claim 8, wherein the columnar base material has a shape of a polygonal prism or a cylinder, and an outer contour of the connection seat is polygonal or circular.

10. The skin treatment element of claim 9, wherein the columnar base material has a shape of a quadrangular prism, a hexagonal prism, or an octagonal prism, and the outer contour of the connection seat is rectangular, hexagonal, or octagonal.

11. The skin treatment element of claim 8, wherein the columnar base material is etched by using the chemical anisotropic etching process along the crystal plane orientation to form an etched plane.

12. The skin treatment element of claim 11, wherein a treatment unit of the treatment units is provided with at least two etched planes, and the at least two etched planes are formed between the connection seat and the treatment end.

13. The skin treatment element of claim 12, wherein the treatment end comprises a second blade portion, the second blade portion and the first blade portion are distributed at an edge of a same etched plane, and the first blade portion and the second blade portion are disconnected.

14. The skin treatment element of claim 12, wherein the treatment end comprises a second blade portion, the second blade portion is connected end-to-end to the first blade portion, and an included angle is formed between the second blade portion and the first blade portion.

15. The skin treatment element of claim 1, wherein the treatment units are made of at least one of a monocrystalline silicon material, a polymer material, or a metal material.

16. The skin treatment element of claim 1, wherein the treatment units are arranged on one side surface of the substrate in a specific array.

17. The skin treatment element of claim 16, wherein the treatment units are arranged on the substrate in a grid-like array, a concentric circular array, or a honeycomb array.

18. The skin treatment element of claim 16, wherein a spacing between two adjacent treatment units is not less than 0.1 mm and not greater than 5 mm.

19. The skin treatment element of claim 1, wherein a treatment unit of the treatment units has a height of not less than 10 nanometers and not greater than 1 millimeter.
